(19) Europäisches Patentamt
European Patent Office
Office européen des brevets

(11) Veröffentlichungsnummer : **0 010 130**
**B1**

(12) **EUROPÄISCHE PATENTSCHRIFT**

(45) Veröffentlichungstag der Patentschrift : **23.12.81**

(51) Int. Cl.³ : **C 07 F 7/10**, A 61 K 31/695

(21) Anmeldenummer : **79102963.0**

(22) Anmeldetag : **20.08.79**

(54) **Sila-substituierte 1,4-Dihydropyridinderivate, Verfahren zu ihrer Herstellung, Arzneimittel und Verfahren zu deren Herstellung.**

(30) Priorität : **28.08.78 DE 2837477**

(43) Veröffentlichungstag der Anmeldung : **30.04.80 (Patentblatt 80/09)**

(45) Bekanntmachung des Hinweises auf die Patenterteilung : **23.12.81 Patentblatt 81/51**

(84) Benannte Vertragsstaaten : **AT BE CH DE FR GB IT NL SE**

(56) Entgegenhaltungen :
**DE - A - 1 545 745**
**DE - A - 2 117 571**
**US - A - 3 781 291**
**US - A - 3 816 439**

(73) Patentinhaber : **BAYER AG**
**Zentralbereich Patente, Marken und Lizenzen**
**D-5090 Leverkusen 1, Bayerwerk (DE)**

(72) Erfinder : **Tacke, Reinhold, Dr.**
**Dorfstrasse 1a**
**D-3300 Braunschweig (DE)**
Erfinder : **Bentlage, Anke**
**Vogelsang 107**
**D-3300 Braunschweig (DE)**
Erfinder : **Towart, Robertson, Dr.**
**Claudiusweg 9**
**D-5600 Wuppertal 1 (DE)**
Erfinder : **Vater, Wulf, Dr.**
**Mechendahlerstrasse 23**
**D-5090 Leverkusen 3 (DE)**

**EP 0 010 130 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

# Sila-substituierte 1,4-Dihydropyridin-Derivate, Verfahren zu ihrer Herstellung, Arzneimittel und Verfahren zu deren Herstellung

Die Erfindung betrifft neue sila-substituierte 1,4-Dihydropyridin-derivate, mehrere Verfahren zu ihrer Herstellung, diese enthaltende Arzneimittel, insbesondere Kreislaufbeeinflussende Arzneimittel sowie Verfahren zu ihrer Herstellung.

Es ist bereits bekannt geworden, daß 1,4-Dihydropyridinderivate wertvolle pharmakologische Eigenschaften besitzen (vergl. DT-OS 2 117 571 und F. Bossert et al., Naturwissenschaften *58*, 578 (1971)). Weiterhin ist bekannt, daß Silicium auch biologisch eine wichtige Rolle spielt und z.B. in Form von Kieselsäureestern an Kohlenhydrate, Steroide und Fette gebunden im Körper von höheren Lebewesen vorkommt (vergl. M.G. Voronkov et al., Silicium und Leben, Akademie-Verlag, Berlin 1975 ; G. Bendz und I. Lindquist (Herausgeber) Biochemistry of Silicon and Related Problems, Plenum Press, New York 1978). Siliciumhaltige 1,4-Dihydropyridin-Derivate sind bisher noch nicht beschrieben worden.

Die vorliegende Erfindung betrifft neue sila-substituierte 1,4-Dihydropyridin-Derivate der allgemeinen Formel I

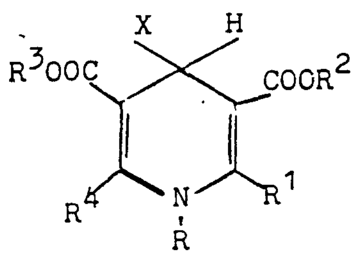

(I)

in welcher

R für Wasserstoff, Alkyl mit 1 bis 4 Kohlenstoffatomen oder Benzyl steht,

$R^1$ und $R^4$ gleich oder verschieden sind und für Wasserstoff, Alkyl mit 1 bis 4 Kohlenstoffatomen oder Trifluormethyl stehen,

$R^2$ und $R^3$ gleich oder verschieden sind und für den Rest

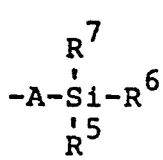

stehen, wobei

A für gegebenenfalls durch Alkyl mit 1 bis 4 Kohlenstoffatomen substituiertes Alkylen mit 1 bis 6 Kohlenstoffatomen steht und

$R^5$, $R^6$ und $R^7$ gleich oder verschieden sind und jeweils für Alkyl mit 1 bis 4 Kohlenstoffatomen, Cycloalkyl mit 3 bis 7 Kohlenstoffatomen oder Phenyl stehen, wobei diese Reste ihrerseits gegebenenfalls substituiert sind durch Alkyl oder Alkoxy mit je 1 bis 2 Kohlenstoffatomen, Halogen, insbesondere Fluor, Chlor oder Brom und Trifluormethyl,

oder

einer der Substituenten $R^2$ oder $R^3$ für gradkettiges oder verzweigtes Alkyl, Cycloalkyl, Alkenyl oder Alkinyl mit bis zu 6 Kohlenstoffatomen steht, wobei der Alkylrest seinerseits gegebenenfalls substituiert ist durch Alkoxy mit 1 bis 4 Kohlenstoffatomen, Halogen, insbesondere Fluor, Chlor, Brom, oder durch einen Phenylrest, der seinerseits 1 oder 2 Substituenten aus der Gruppe Trifluormethyl, Nitro, Halogen, insbesondere Fluor, Chlor, Brom, Alkyl und Alkoxy mit je 1 bis 4 Kohlenstoffatomen tragen kann

oder

einer der Substituenten $R^2$ und $R^3$ für Alkyl mit 1 bis 4 Kohlenstoffatomen steht, welches substituiert ist durch die Gruppe

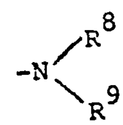

wobei $R^8$ und $R^9$ gleich oder verschieden sind und jeweils für Wasserstoff, Alkyl mit 1 bis 4 Kohlenstoffatomen, Phenyl oder Benzyl stehen und

X für Alkyl mit 1 bis 4 Kohlenstoffatomen, Cycloalkyl mit 3 bis 7 Kohlenstoffatomen, Cycloalkenyl mit 5 bis 7 Kohlenstoffatomen, Phenylalkyl mit 1 bis 3 Kohlenstoffatomen in der Alkylkette, Phenyl oder Naphthyl steht, wobei der Phenylrest gegebenenfalls substituiert ist durch 1, 2 oder 3 gleiche oder verschiedene Substituenten aus der Gruppe Nitro, Cyan, Halogen, insbesondere Fluor, Chlor, Brom, Trifluormethyl, Amino, Trifluormethoxy, Alkyl, Alkoxy, Alkylmercapto oder Alkylsulfonyl mit je 1 bis 4 Kohlenstoffatomen in den genannten Alkyl und Alkoxyresten,

oder für einen gegebenenfalls durch Halogen, insbesondere Fluor, Chlor, Brom, Alkyl mit 1 bis 4 Kohlenstoffatomen oder Alkoxy mit 1 bis 4 Kohlenstoffatomen substituierten Chinolyl-, Isochinolyl-, Pyridyl-, Pyrimidyl-, Thienyl-, Furyl- oder Pyrrylrest steht.

Es wurde gefunden, daß man die sila-substituierten 1,4-Dihydropyridin-Derivate der allgemeinen Formel I erhält, wenn man

a) Aldehyde der allgemeinen Formel II

$$X{-}CHO \qquad (II)$$

in welcher X die obenangegebene Bedeutung hat, mit β-Dicarbonylverbindungen der allgemeinen Formel III

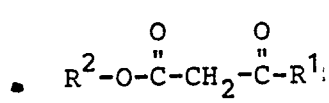

(III)

in welcher $R^1$ und $R^2$ die obenangegebene Bedeutung haben, und mit Enaminen der allgemeinen Formel IV

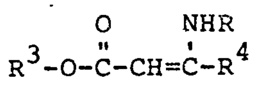 (IV)

in welcher R, $R^3$ und $R^4$ die obenangegebene Bedeutung haben, gegebenenfalls in Gegenwart inerter organischer Lösungsmittel bei erhöhter Temperatur umsetzt oder

b) Ylidenverbindungen der allgemeinen Formel V

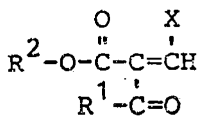 (V)

in welcher $R^1$, $R^2$ und X die obenangegebene Bedeutung haben, mit Enaminen der obenangegebenen Formel IV gegebenenfalls in Gegenwart von inerten organischen Lösungsmitteln umsetzt oder

c) zur Herstellung symmetrischer sila-substituierter 1,4-Dihydropyridin-Derivate Aldehyde der oben angegebenen Formel II mit der zweifachmolaren Menge siliciumhaltiger β-Dicarbonylverbindungen der allgemeinen Formel III, in welcher der siliciumhaltige Rest $R^2$ und $R^1$ die oben angegebene Bedeutung haben,

und mit Aminderivaten der allgemeinen Formel VI

$$H_2N—R \qquad (VI)$$

in welcher R die obenangegebene Bedeutung hat, gegebenenfalls in Gegenwart inerter organischer Lösungsmittel umsetzt.

Bemerkenswerterweise zeigen die erfindungsgemäßen sila-substituierten 1,4-Dihydropyridin-Derivate der allgemeinen Formel I starke kreislaufbeeinflussende Wirkungen. Insbesondere hemmen die erfindungsgemäßen Verbindungen die Kontraktilität der glatten Gefäßmuskulatur. Sie bewirken eine deutliche und langanhaltende Erweiterung der Coronargefäße und beeinflussen den Herzstoffwechsel im Sinne einer Energieersparnis. Weiterhin senken die Verbindungen den Blutdruck und können als antihypertensive Mittel verwendet werden. Die gefäßspasmolytische Wirkung der sila-substituierten 1,4-Dihydropyridin-Derivate kann im gesamten Gefäßsystem stattfinden oder sich isoliert in speziellen Gefäßgebieten wie z.B. im peripheren Bereich, im Coronarbereich oder im cerebralen Bereich auswirken.

Bei Kenntnis des Standes der Technik konnte nicht erwartet werden, daß die erfindungsgemäßen Verbindungen so vorteilhafte kreislaufbeeinflussende Wirkungen besitzen. In der Publikation von R. Tacke und U. Wannagat, Topics in Current Chemistry *84,* 1-75 (1979) wird darauf hingewiesen, daß die biologischen Wirkungen beim Austausch eines Kohlenstoffatoms durch ein Siliciumatom letztlich nicht vorhersehbar sind. Gleichzeitig ist bekannt, daß bei Dihydropyridin-Derivaten die Esterfunktion eine wesentliche Rolle für die Strukturwirkungsbeziehungen spielen, wie aus F. Bossert et al., Arzneimittel-Forschung *29,* 226-229 (1979) hervorgeht. Durch diese Literaturstellen wird ersichtlich, daß eine vorteilhaftere Wirkung der Silicium-haltigen-Verbindung nicht erwartet werden konnte.

Die neuen sila-substituierten 1,4-Dihydropyridin-Derivate können in bekannter Weise in die üblichen Formulierungen überführt werden, wie Tabletten, Kapseln, Dragees, Pillen, Granulate, Aerosole, Sirupe, Emulsionen, Suspensionen und Lösungen, unter Verwendung inerter, nichttoxischer, pharmazeutisch geeigneter Trägersubstanzen oder Lösungsmittel. Hierbei soll die therapeutischwirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Verbindungen mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt:

Wasser, nichttoxische organische Lösungsmittel, wie Paraffine (z.B. Erfölfraktionen), pflanzliche Öle (z.B. Erdnuß-/Sesamöl), Alkohole (z.B. Aethylalkohol, Glycerin), Glykole (z.B. Propylenglykol, Polyäthylenglykol); feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker); Emulgiermittel, wie nichtionogene und anionische Emulgatoren (z.B. Polyoxyäthylen-Fettsäure-Ester, Polyoxyäthylen-Fettalkohol-Äther, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös.

Im Falle der oralen Anwendung können Tabletten selbstverständlich außer den genannten Trägerstoffen auch Zusätze wie Natriumcitrat und Calciumcarbonat zusammen mit verschiedenen Zuschlagstoffen, wie Stärke, vorzugsweise Kartoffelstärke, Gelantine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixiere, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,002 bis 1 mg/kg Körpergewicht pro Tag zur Erzielung wirksamer Ergebnisse zu verabreichen. Bei oraler Applikation beträgt die Dosierung etwa 0,05 bis 50 mg/kg, vorzugsweise 0,5 bis 10 mg/kg Körpergewicht pro Tag.

Trotzdem kann es gegebenenfalls erforderlich sein, von den genannten Mengen abzuweichen, und zwar in Abhängigkeit von der Art des Applikationsweges, aber auch aufgrund der Tierart und deren individuellen Verhalten gegenüber dem Medikament bzw. der Art von dessen Formulierung und dem Zeitpunkt bzw. Intervall, zu welchem die Verabreichung erfolgt. So kann es in wenigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hier auch die obigen Ausführungen.

Von besonderem Interesse sind sila-substituierte 1,4-Dihydropyridin-Derivate der allgemeinen Formel I, in welcher

R für Wasserstoff oder Methyl steht,

$R^1$ und $R^4$ für Methyl stehen,

$R^2$ und $R^3$ gleich oder verschieden sind und für den Rest

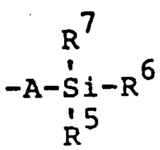

stehen, wobei

A für gegebenenfalls durch Methyl substituiertes Alkylen mit 1 bis 6 Kohlenstoffatomen steht und

$R^5$, $R^6$ und $R^7$ gleich oder verschieden sind und jeweils für Alkyl mit 1 bis 4 Kohlenstoffatomen, Cycloalkyl mit 3 bis 7 Kohlenstoffatomen oder Phenyl stehen, wobei diese Reste ihrerseits gegebenenfalls substituiert sind durch Alkyl oder Alkoxy mit je 1 bis 2 Kohlenstoffatomen, Halogen, insbesondere Fluor, Chlor oder Brom, und Trifluormethyl,

oder

einer der Substituenten $R^2$ oder $R^3$ für gradkettiges oder verzweigtes Alkyl, Cycloalkyl, Alkenyl oder Alkinyl mit bis zu 6 Kohlenstoffatomen steht, wobei der Alkylrest seinerseits gegebenenfalls substituiert ist durch Alkoxy mit 1 bis 4 Kohlenstoffatomen, Halogen, insbesondere Fluor, Chlor, Brom, oder durch einen Phenylrest, der seinerseits 1 oder 2 Substituenten aus der Gruppe Trifluormethyl, Nitro, Halogen, insbesondere Fluor, Chlor, Brom, Alkyl und Alkoxy mit je 1 bis 4 Kohlenstoffatomen tragen kann

oder

einer der Substituierten $R^2$ und $R^3$ für Alkyl mit 1 bis 4 Kohlenstoffatomen steht, welches substituiert ist durch die Gruppe

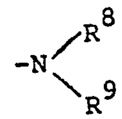

wobei $R^8$ und $R^9$ gleich oder verschieden sind und jeweils für Wasserstoff, Alkyl mit 1 bis 4 Kohlenstoffatomen, Phenyl oder Benzyl stehen und

X für Phenyl oder Naphthyl steht, wobei der Phenylrest gegebenenfalls substituiert ist durch 1, 2 oder 3 gleiche oder verschiedene Substituenten aus der Gruppe Nitro, Cyan, Halogen, insbesondere Fluor, Chlor, Brom, Trifluormethyl, Amino, Trifluormethoxy, Alkyl, Alkoxy, Alkylmercapto, oder Alkylsulfonyl mit je 1 bis 4 Kohlenstoffatomen, oder für einen gegebenenfalls durch Halogen, insbesondere Fluor, Chlor, Brom, Alkyl mit 1 bis 4 Kohlenstoffatomen oder Alkoxy mit 1 bis 4 Kohlenstoffatomen substituierten Chinolyl-, Isochinolyl-, Pyridyl, Pyrimidyl-, Thienyl-, Furyl- oder Pyrrylrest steht.

Bei der Durchführung des erfindungsgemäßen Verfahrens sind die als Ausführungsprodukte einsetzbaren Aldehyde der allgemeinen Formel II bekannt oder können nach bekannten Methoden hergestellt werden (E. Mosettig, Org. reactions VIII, 218 ff. (1954)). Als Beispiele seien genannt :

Aldehyde

2-, 3- oder 4-Nitrobenzaldehyd, 2,4- oder 2,6-Dinitrobenzaldehyd, 2-Nitro-6-brombenzaldehyd, 2-Nitro-3-methoxybenzaldehyd, 2-, oder 3-Chlorbenzaldehyd, 3-(Trifluormethyl) benzaldehyd, 2-Nitro-3-methoxy-6-chlorbenzaldehyd, 2-Nitro-4-methoxybenzaldehyd, 3-Nitro-6-chlorbenzaldehyd, 2-, 3- oder 4-Cyanbenzaldehyd, α-, β- oder γ-Pyridinaldehyd, 6-Methylpyridin-2-aldehyd, Pyrimidin-5-aldehyd, 4,6-Dimethoxypyrimidin-5-aldehyd, 2-Methylmercaptobenzaldehyd, 2-Methylsulfonylbenzaldehyd, 2-Methylsulfinyl-benzaldehyd, 1- oder 2-Naphthaldehyd, 5-Brom-1-naphthaldehyd, 2-Äthoxy-1-naphthaldehyd, 4-Methyl-1-naphthaldehyd, Chinolin-2-, 3-, 4-, 5-, 6-, 7-oder 8-aldehyd, Isochinolin-1- oder 3-aldehyd, Furan-2-aldehyd, Thiophen-2-aldehyd, Pyrrol-2-aldehyd.

Die erfindungsgemäß einsetzbaren siliciumfreien und siliciumhaltigen β-Dicarbonylverbindungen der allgemeinen Formel III sind teilweise bekannt oder können nach bekannten Methoden hergestellt werden. Die siliciumhaltigen Verbindungen der allgemeinen Formel III lassen sich in Analogie zu den siliciumfreien β-Dicarbonylverbindungen darstellen [V.F. Mironov, V.P. Kozyukov und V.D. Sheludyakov, Zh. Obsh. Khim. *37* 1915 (1967)].

Als Beispiele seien genannt :

β-Dicarbonylverbindungen

Acetessigsäure-methylester, Acetessigsäure-äthylester, Acetessigsäurepropylester, Acetessigsäure-propargylester, Acetessigsäure-allylester, Acetessigsäure-(α- oder β) methoxy-äthylester, Acetessigsäure-(α- oder β)-äthoxyäthylester, Formylessigsäure-äthylester, Acetessigsäure-cyclopentylester, Acetessigsäure-neopentylester, Acetessigsäure-(3,3-dimethyl-butyl) ester, Acetessigsäure-(trimethylsilyl-methyl) ester, Acetessigsäure-(2-trimethylsilyl-äthyl) ester, Acetessigsäure-(3-trimethylsilyl-propyl) ester, Acetessigsäure-(2-triäthylsilyl-äthyl)-ester, Acetessigsäure-(äthyldimethylsilyl-methyl) ester, Acetessigsäure-(phenyldimethylsilyl-methyl) ester, Acetessigsäure-(p-fluorphenyldimethylsilyl-methyl) ester.

Die erfindungsgemäß einsetzbaren siliciumfreien Enamine der allgemeinen Formel IV sind bereits bekannt oder können nach bekannten Methoden hergestellt werden. Die siliciumhaltigen Enamine der allgemeinen Formel IV sind bisher nicht bekannt, lassen sich aber in Analogie zu den siliciumfreien Enaminen aus den entsprechenden β-Dicarbonylverbindungen der allgemeinen Formel III und Aminen der allgemeinen Formel VI herstellen. Als Beispiele seien genannt :

Enamine

β-Aminocrotonsäure-methylester, β-Aminocrotonsäure-äthylester, β-Aminocrotonsäure-propylester, β-Aminocrotonsäure-isopropylester, β-Aminocrotonsäure-neopentylester, β-Aminocrotonsäure-(3,3-dimethylbutyl) ester, β-Aminocrotonsäure-(trimethylsilyl-methyl) ester, β-Aminocrotonsäure-trimethylsilyl-methyl) ester, β-Aminocrotonsäure-(triäthylsilyl-methyl) ester, β-Aminocrotonsäure-(äthyldimethylsilyl-methyl) ester, β-Aminocrotonsäure-(phenyldimethylsilylmethyl) ester.

Die erfindungsgemäß einsetzbaren siliciumfreien Ylidenverbindungen der allgemeinen Formel V sind bereits bekannt oder können nach bekannten Methoden hergestellt werden. Die erfindungsgemäß einsetzbaren siliciumhaltigen Ylidenverbindungen der allgemeinen Formel V sind dagegen bisher nicht bekannt, können aber in Analogie zu den siliciumfreien Verbindungen der allgemeinen Formel V aus Aldehyden der allgemeinen Formel II und aus den entsprechenden β-Dicarbonylverbindungen der algemeinen Formel III durch Knoevenagel-Kondensation hergestellt werden. Als Beispiele seien genannt :

Ylidenverbindungen

2-(2-Nitrobenzyliden)-acetessigsäure-methylester,

2-(3-Nitrobenzyliden)-acetessigsäure-methylester,

2-(4-Nitrobenzyliden) acetessigsäure-methylester,

2-(3-Nitrobenzyliden)-acetessigsäure-neopentylester,

2-(3-Nitrobenzyliden)-acetessigsäure-(3,3-dimethylbutyl) ester, 2-(3-Nitrobenzyliden)-acetessigsäure-(propin-2-yl) ester, 2-(3-Nitrobenzyliden) acetessigsäure-isopropylester, 2-(3-Nitrobenzyliden)-acetessigsäure-(2-methoxyäthyl) ester, 2-(3-Nitrobenzyliden)-acetessigsäure-(trimethylsilyl-methyl) ester, 2-(3-Nitrobenzyliden)-acetessigsäure-(äthyldimethyl) ester, 2-(3-Chlorbenzyliden)-acetessigsäure-(trimethylsilylmethyl) ester.

Die erfindungsgemäßen einsetzbaren Aminderivate der allgemeinen Formel VI sind bereits bekannt ; als Beispiele seien genannt :

Ammoniak, Methylamin, Äthylamin, Propylamin, Isopropylamin, Butylamin, Isobutylamin, Benzylamin.

Als Verdünnungsmittel kommen Wasser und alle inerten organischen Lösungsmittel in Frage. Hierzu gehören vorzugsweise Alkohole wie Äthanol, Methanol, Äther wie Dioxan, Diäthyläther oder Eisessig, Dimethylformamid, Dimethylsulfoxid, Acetonitril, usw.

Die Reaktionstemperaturen können in einem größeren Bereich variiert werden. Im allgemeinen arbeitet man bei 80-90 °C. Die bevorzugte Ausführung besteht darin, daß man die Umsetzungen in siedendem Äthanol vornimmt.

Die Umsetzung kann bei Normaldruck, aber auch bei erhöhtem Druck durchgeführt werden. Im allgemeinen arbeitet man bei Normaldruck.

Bei der Durchführung der erfindungsgemäßen Verfahrensvarianten werden die an der Reaktion beteiligten Stoffe jeweils in etwa stöchiometrischen Mengen eingesetzt. Das verwendete Amin der allgemeinen Formel VI bzw. dessen Salz wird vorzugsweise im Überschuß (1,5-2 fach molare Menge) zugegeben.

Als neue Wirkstoffe seien im einzelnen genannt :

2,6-Dimethyl-4-(2-nitrophenyl)-1,4-dihydropyridin-3,5-dicarbonsäure-3-äthylester-5-(trimethylsilyl-methyl)-ester

2,6-Dimethyl-4-(3-nitrophenyl)-1,4-dihydropyridin-3,5-dicarbonsäure-3-äthylester-5-(trimethylsilyl-methyl)-ester

2,6-Dimethyl-4-(2-nitrophenyl)-1,4-dihydropyridin-3,5-dicarbonsäure-3-methylester-5-(2-trimethylsilyl-äthyl)-ester

2,6-Dimethyl-4-(3-nitrophenyl)-1,4-dihydropyridin-3,5-dicarbonsäure-3-methylester-5-(2-trimethylsilyl-äthyl)-ester

2,6-Dimethyl-4-(4-nitrophenyl-1,4-dihydropyridin-3,5-dicarbonsäure-3-methylester-5-(2-trimethylsilyl-äthyl)-ester

2,6-Dimethyl-4-(2-nitrophenyl)-1,4-dihydropyridin-3,5-dicarbonsäure-3-methylester-5-(dimethylphenylsilyl-methyl)-ester

2,6-Dimethyl-4-(3-nitrophenyl)-1,4-dihydropyridin-3,5-dicarbonsäure-3-methylester-5-(dimethylphenylsilyl-methyl)-ester

2,6-Dimethyl-4-(2-chlorphenyl)-1,4-dihydropyridin-3,5-dicarbonsäure-3-methylester-5-(dimethylphenylsilyl-methyl) ester

2,6-Dimethyl-4-(3-nitrophenyl)-1,4-dihydropyridin-3,5-dicarbonsäure-3-(trimethylsilyl-methyl) ester-5-(2-trimethylsilyl-äthyl) ester

2,6-Dimethyl-4-(2-chlorphenyl)-1,4-dihydropyridin-3,5-dicarbonsäure-3-methylester-5-(trimethylsilyl-methyl)-ester

1,2,6-Trimethyl-4-(2-nitrophenyl)-1,4-dihydropyridin-3,5-dicarbonsäure-3-methylester-5-(trimethylsilyl-methyl) ester

1,2,6-Trimethyl-4-(3-nitrophenyl)-1,4-dihydropyridin-3,5-dicarbonsäure-3-methylester-5-(trimethylsilyl-methyl) ester

Herstellungsbeispiele

Beispiel 1

2,6-Dimethyl-4-(3-nitrophenyl)-1,4-dihydropyridin-3,5-dicarbonsäure-bis(trimethylsilyl-methyl) ester

18,2 g Acetessigsäure-(trimethylsilyl-methyl) ester, 7,3 g 3-Nitrobenzaldehyd und 5,5 ml konz. Ammoniak werden in 30 ml 96 proz. Äthanol 20 Stunden zum Sieden erhitzt. Nach dem Abkühlen saugt man ab, trocknet und erhält 16,9 g gelber Kristalle vom Fp. 159 °C (Äthanol).

Auf die gleiche Weise erhält man auch 20 Stündigem Kochen in 20 ml 96 proz. Äthanol aus 11,3 g Acetessigsäure-(trimethylsilyl-methyl) ester, 3,3 ml konz. Ammoniak

a) und 4,5 g 2-Nitrobenzaldehyd 8,1 g 2,6-Dimethyl-4-(2-nitrophenyl)-1,4-dihydropyridin-3,5-dicarbonsäure-bis(trimethylsilyl-methyl) ester, Fp. 157 °C (Äthanol).

b) und 3,18 g Benzaldehyd 6,2 g 2,6-Dimethyl-4-phenyl-1,4-dihydropyridin-3,5-dicarbonsäure-bis(trimethylsilyl-methyl)-ester, Fp. 123 °C (Äther/Petroläther).

c) und 4,22 g 3-Chlorbenzaldehyd 6,7 g 2,6-Dimethyl-4-(3-chlorphenyl)-1,4-dihydropyridin-3,5-dicarbonsäure-bis(trimethylsilyl-methyl) ester, Fp. 129 °C (Äther/Petroläther).

d) und 4,22 g 2-Chlorbenzaldehyd 6,9 g 2,6-Dimethyl-4-(2-chlorphenyl)-1,4-dihydropyridin-3,5-dicarbonsäure-bis(trimethylsilyl-methyl) ester, Fp. 128 °C (Äther/Petroläther).

e) und 3,21 g Pyridin-3-aldehyd 11,3 g 2,6-Dimethyl-4-(3-pyridyl)-1,4-dihydropyridin-3,5-dicarbonsäure-bis(trimethylsilyl-methyl) ester, Fp. 149 °C (Äthanol/Äther)

f) und 3,21 g Pyridin-2-aldehyd 8,1 g 2,6-Dimethyl-4-(2-pyridyl)-1,4-dihydropyridin-3,5-dicarbonsäure-bis-(trimethylsilyl-methyl) ester, Fp. 205 °C (Äthanol/Methanol)

Beispiel 2

2,6-Dimethyl-4-(3-nitrophenyl)-1,4-dihydropyridin-3,5-dicarbonsäure-bis(dimethylphenylsilyl-methyl) ester

Man erhitzt 10,0 g Acetessigsäure-(dimethylphenylsilyl-methyl) ester, 3,0 g 3-Nitrobenzaldehyd und 2,2 ml konz. Ammoniak in 15 ml 96 proz. Äthanol 20 Stunden unter Rückfluß. Nach Kühlen und Absaugen erhält man 11,0 g gelber Kristalle vom Fp. 125 °C (Äthanol).

Beispiel 3

2,6-Dimethyl-4-(3-nitrophenyl)-1,4-dihydropyridin-3,5-dicarbonsäure-bis(2-trimethylsilyl-äthyl) ester

Nach 20 stündigem Kochen von 5,0 g Acetessigsäure-(2-trimethylsilyläthyl) ester, 1,9 g 3-Nitrobenzaldehyd und 1,5 ml konz. Ammoniak in 10 ml 96 proz. Äthanol, anschließendem Kühlen und Absaugen erhält man 3,5 g hellgelber Kristalle vom Fp. 120 °C (Äthanol).

Beispiel 4

2,6-Dimethyl-4-(3-nitrophenyl)-1,4-dihydropyridin-3,5-dicarbonsäure-3-neopentylester-5-(trimethylsilyl-methyl)-ester

Nach 17 stündigem Erhitzen von 5,6 g β-Aminocrotonsäure-(trimethylsilyl-methyl) ester, 5,2 g Acetessigsäure-neopentylester und 4,5 g 3-Nitrobenzaldehyd in 20 ml 96 proz. Äthanol läßt man abkühlen, saugt ab und erhält 10,4 g gelber Kristalle vom Fp. 176 °C (Äthanol).

Beispiel 5

2,6-Dimethyl-4-(2-nitrophenyl)-1,4-dihydropyridin-3,5-dicarbonsäure-3-methylester-5-(trimethylsilyl-methyl)-ester

6,44 g 2-(2-Nitrobenzyliden)-acetessigsäure-methylester und 4,84 g β-Aminocrotonsäure-(trimethylsilyl-methyl)-ester werden in 17 ml 96 proz. Äthanol 20 Stunden unter Rückfluß erhitzt. Das Lösungsmittel wird anschließend i. Vak. abgezogen und der Rückstand aus Äthanol/Äther (1 : 1) kristallisiert. Man erhält 7,5 g tiefgelber Kristalle vom Fp. 156 °C (Äthanol/Äther).

Auf die gleiche Weise erhält man nach 20 stündigem Kochen in 20 ml 96 proz. Äthanol aus 5,62 g β-Aminocrotonsäure-(trimethylsilyl-methyl) ester

a) und 7,48 g 2-(3-Nitrobenzyliden)-acetessigsäure-methylester 10,6 g 2,6-Dimethyl-4-(3-nitrophenyl)-1,4-dihydropyridin-3,5-dicarbonsäure-3-methylester-5-(trimethylsilyl-methyl) ester, Fp. 120 °C (Äther/Petroläther).

b) und 7,48 g 2-(4-Nitrobenzyliden)-acetessigsäure-methylester 11,3 g 2,6-Dimethyl-4-(4-nitrophenyl)-1,4-dihydropyridin-3,5-dicarbonsäure-3-methylester-5-(trimethylsilyl-methyl) ester, Fp. 137 °C (Methanol).

Beispiel 6

2,6-Dimethyl-4-(3-nitrophenyl)-1,4-dihydropyridin-3,5-dicarbonsäure-3-(propin-2-yl) ester-5-(trimethylsilyl-methyl) ester

Nach 20 stündigem Erhitzen von 5,62 g β-Aminocrotonsäure-(trimethylsilyl-methyl) ester, 8,20 g 2-(3-Nitrobenzyliden)-acetessigsäure-(propin-2-yl) ester und 20 ml 96 proz. Äthanol läßt man abkühlen und erhält 14,25 g hellgelber Kristalle. Fp. 148 °C (Äthanol).

Beispiel 7

2,6-Dimethyl-4-(3-nitrophenyl)-1,4-dihydropyridin-3,5-dicarbonsäure-3-isopropylester-5-(trimethylsilyl-methyl)-ester

Man erhitzt 8,32 g 2-(3-Nitrobenzyliden)-acetessigsäure-isopropylester und 5,62 g β-Aminocrotonsäure-(trimethylsilyl-methyl) ester für 20 Stunden in 20 ml 96 proz. Äthanol, läßt abkühlen und erhält 11,8 g gelber Kristalle vom Fp. 121 °C (Äthanol/Äther).

Beispiel 8

2,6-Dimethyl-4-(3-nitrophenyl)-1,4-dihydropyridin-3,5-dicarbonsäure-3-(2-methoxyäthyl) ester-5-(trimethylsilyl-methyl) ester

8,80 g 2-(3-Nitrobenzyliden)-acetessigsäure-(2-methoxyäthyl) ester und 5,62 g β-Aminocrotonsäure-(trimethylsilyl-methyl) ester werden 20 Stunden in 20 ml 96 proz.

Äthanol zum Rückfluß erhitzt. Anschließend zieht man das Lösungsmittel ab und kristallisiert aus Äther/Petroläther (1 : 1). Durch Filtration erhält man 11,5 g gelber Kristalle vom Fp. 92 °C (Äther/Petroläther).

**Ansprüche**

1. Sila-substituierte 1,4-Dihydropyridin-Derivate der allgemeinen Formel I

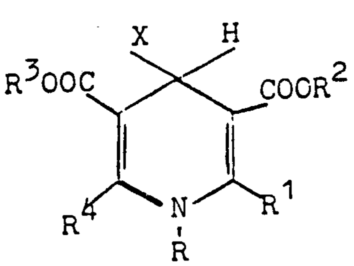

(I)

in welcher

R für Wasserstoff, Alkyl mit 1 bis 4 Kohlenstoffatomen oder Benzyl steht,

$R^1$ und $R^4$ gleich oder verschieden sind und für Wasserstoff, Alkyl mit 1 bis 4 Kohlenstoffatomen oder Trifluormethyl stehen,

$R^2$ und $R^3$ gleich oder verschieden sind und für den Rest

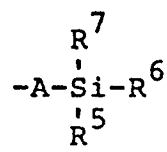

stehen, wobei

A für gegebenenfalls durch Alkyl mit 1 bis 4 Kohlenstoffatomen substituiertes Alkylen mit 1 bis 6 Kohlenstoffatomen steht und

$R^5$, $R^6$ und $R^7$ gleich oder verschieden sind und jeweils für Alkyl mit 1 bis 4 Kohlenstoffatomen, Cycloalkyl mit 3 bis 7 Kohlenstoffatomen oder Phenyl stehen, wobei diese Reste ihrerseits gegebenenfalls substituiert sind durch Alkyl oder Alkoxy mit je 1 bis 2 Kohlenstoffatomen, Halogen, insbesondere Fluor, Chlor oder Brom und Trifluormethyl,

oder

einer der Substituenten $R^2$ oder $R^3$ für gradkettiges oder verzweigtes Alkyl, Cycloalkyl, Alkenyl oder Alkinyl mit bis zu 6 Kohlenstoffatomen steht, wobei der Alkylrest seinerseits gegebenenfalls substituiert ist durch Alkoxy mit 1 bis 4 Kohlenstoffatomen, Halogen, insbesondere Fluor, Chlor, Brom, oder durch einen Phenylrest, der seinerseits 1 oder 2 Substituenten aus der Gruppe Trifluormethyl, Nitro, Halogen, insbesondere Fluor, Chlor, Brom, Alkyl und Alkoxy mit je 1 bis 4 Kohlenstoffatomen tragen kann

oder

einer der Substituenten $R^2$ und $R^3$ für Alkyl mit 1 bis 4 Kohlenstoffatomen steht, welches substituiert ist durch die Gruppe

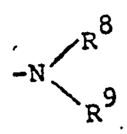

wobei

$R^8$ und $R^9$ gleich oder verschieden sind und jeweils für Wasserstoff, Alkyl mit 1 bis 4 Kohlenstoffatomen, Phenyl oder Benzyl stehen und

X für Alkyl mit 1 bis 4 Kohlenstoffatomen, Cycloalkyl mit 3 bis 7 Kohlenstoffatomen, Cycloalkenyl mit 5 bis 7 Kohlenstoffatomen, Phenylalkyl mit 1 bis 3 Kohlenstoffatomen in der Alkylkette, Phenyl oder Naphthyl steht, wobei der Phenylrest gegebenenfalls substituiert ist durch 1, 2 oder 3 gleiche oder verschiedene Substituenten aus der Gruppe Nitro, Cyan, Halogen, insbesondere Fluor, Chlor, Brom, Trifluormethyl, Amino, Trifluormethoxy, Alkyl, Alkoxy, Alkylmercapto oder Alkylsulfonyl mit je 1 bis 4 Kohlenstoffatomen in den genannten Alkyl und Alkoxyresten,

oder für einen gegebenenfalls durch Halogen, insbesondere Fluor, Chlor, Brom, Alkyl mit 1 bis Kohlenstoffatomen oder Alkoxy mit 1 bis 4 Kohlenstoffatomen substituierten Chinolyl-, Isochinolyl-, Pyridyl-, Pyrimidyl-, Thienyl-, Furyl- oder Pyrrylrest steht.

2. Verbindungen der allgemeinen Formel I gemäß Anspruch 1
in welcher
R für Wasserstoff oder Methyl steht,
$R^1$ und $R^4$ für Methyl stehen,
$R^2$ und $R^3$ gleich oder verschieden sind und für den Rest

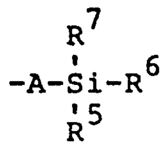

stehen, wobei
A für gegebenenfalls durch Methyl substituiertes Alkylen mit 1 bis 6 Kohlenstoffatomen steht und
$R^5$, $R^6$ und $R^7$ gleich oder verschieden sind und jeweils für Alkyl mit 1 bis 4 Kohlenstoffatomen, Cycloalkyl mit 3 bis 7 Kohlenstoffatomen oder Phenyl stehen, wobei diese Reste ihrerseits gegebenenfalls substituiert sind durch Alkyl oder Alkoxy mit je 1 bis 2 Kohlenstoffatomen, Halogen, insbesondere Fluor, Chlor oder Brom, und Trifluormethyl,
oder
einer der Substituenten $R^2$ oder $R^3$ für gradkettiges oder verzweigtes Alkyl, Cycloalkyl, Alkenyl oder Alkinyl mit bis zu 6 Kohlenstoffatomen steht, wobei der Alkylrest seinerseits gegebenenfalls substituiert ist durch Alkoxy mit 1 bis 4 Kohlenstoffatomen, Halogen, insbesondere Fluor, Chlor, Brom, oder durch einen Phenylrest, der seinerseits 1 oder 2 Substituenten aus der Gruppe Trifluormethyl, Nitro, Halogen, insbesondere Fluor, Chlor, Brom, Alkyl und Alkoxy mit je 1 bis 4 Kohlenstoffatomen tragen kann
oder
einer der Substituierten $R^2$ und $R^3$ für Alkyl mit 1 bis 4 Kohlenstoffatomen steht, welches substituiert ist durch die Gruppe

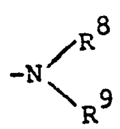

wobei
$R^8$ und $R^9$ gleich oder verschieden sind und jeweils für Wasserstoff, Alkyl mit 1 bis 4 Kohlenstoffatomen, Phenyl oder Benzyl stehen und
X für Phenyl oder Naphthyl steht, wobei der Phenylrest gegebenenfalls substituiert ist durch 1, 2 oder 3 gleiche oder verschiedene Substituenten aus der Gruppe Nitro, Cyan, Halogen, insbesondere Fluor, Chlor, Brom, Trifluormethyl, Amino, Trifluormethoxy, Alkyl, Alkoxy, Alkylmercapto, oder Alkylsulfonyl mit je 1 bis 4 Kohlenstoffatomen, oder für einen gegebenenfalls durch Halogen, insbesondere Fluor, Chlor, Brom, Alkyl mit 1 bis 4 Kohlenstoffatomen oder Alkoxy mit 1 bis 4 Kohlenstoffatomen substituierten Chinolyl-, Isochinolyl-, Pyridyl, Pyrimidyl-, Thienyl-, Furyl- oder Pyrrylrest steht.

3. Verfahren zur Herstellung von sila-substituierten 1,4-Dihydropyridin-Derivaten der allgemeinen Formel I

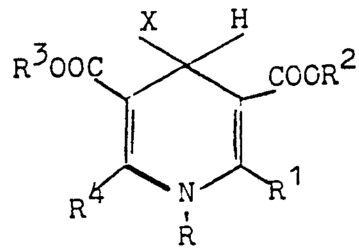

(I)

in welcher
R für Wasserstoff, Alkyl mit 1 bis 4 Kohlenstoffatomen oder Benzyl steht,
$R^1$ und $R^4$ gleich oder verschieden sind und für Wasserstoff, Alkyl mit 1 bis 4 Kohlenstoffatomen oder Trifluormethyl stehen,
$R^2$ und $R^3$ gleich oder verschieden sind und für den Rest

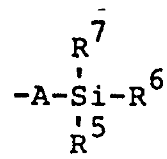

stehen, wobei
A für gegebenenfalls durch Alkyl mit 1 bis 4 Kohlenstoffatomen substituiertes Alkylen mit 1 bis 6 Kohlenstoffatomen steht und
$R^5$, $R^6$ und $R^7$ gleich oder verschieden sind und jeweils für Alkyl mit 1 bis 4 Kohlenstoffatomen, Cycloalkyl mit 3 bis 7 Kohlenstoffatomen oder Phenyl stehen, wobei diese Reste ihrerseits gegebenenfalls substituiert sind durch Alkyl oder Alkoxy mit je 1 bis 2 Kohlenstoffatomen, Halogen, insbesondere Fluor, Chlor oder Brom und Trifluormethyl,
oder
einer der Substituenten $R^2$ oder $R^3$ für gradkettiges oder verzweigtes Alkyl, Cycloalkyl, Alkenyl oder Alkinyl mit bis zu 6 Kohlenstoffatomen steht, wobei der Alkylrest seinerseits gegebenenfalls substituiert ist durch Alkoxy mit 1 bis 4 Kohlenstoffatomen, Halogen, insbesondere Fluor, Chlor, Brom, oder durch einen Phenylrest, der seinerseits 1 oder 2 Substituenten aus der Gruppe Trifluormethyl, Nitro, Halogen, insbesondere Fluor, Chlor, Brom, Alkyl und Alkoxy mit je 1 bis 4 Kohlenstoffatomen tragen kann
oder
einer der Substituenten $R^2$ und $R^3$ für Alkyl mit 1 bis 4 Kohlenstoffatomen steht, welches substituiert ist durch die Gruppe

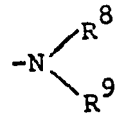

wobei
$R^8$ und $R^9$ gleich oder verschieden sind und

jeweils für Wasserstoff, Alkyl mit 1 bis 4 Kohlenstoffatomen, Phenyl oder Benzyl stehen und

X für Alkyl mit 1 bis 4 Kohlenstoffatomen, Cycloalkyl mit 3 bis 7 Kohlenstoffatomen, Cycloalkenyl mit 5 bis 7 Kohlenstoffatomen, Phenylalkyl mit 1 bis 3 Kohlenstoffatomen in der Alkylkette, Phenyl oder Naphthyl steht, wobei der Phenylrest gegebenenfalls substituiert ist durch 1, 2 oder 3 gleiche oder verschiedene Substituenten aus der Gruppe Nitro, Cyan, Halogen, insbesondere Fluor, Chlor, Brom, Trifluormethyl, Amino, Trifluormethoxy, Alkyl, Alkoxy, Alkylmercapto oder Alkylsulfonyl mit je 1 bis 4 Kohlenstoffatomen in den genannten Alkyl und Alkoxyresten,

oder für einen gegebenenfalls durch Halogen, insbesondere Fluor, Chlor, Brom, Alkyl mit 1 bis 4 Kohlenstoffatomen oder Alkoxy mit 1 bis 4 Kohlenstoffatomen substituierten Chinolyl-, Isochinolyl-, Pyridyl-, Pyrimidyl-, Thienyl-, Furyl- oder Pyrrylrest steht,

dadurch gekennzeichnet, daß man

a) Aldehyde der allgemeinen Formel II

$$X - CHO \qquad (II)$$

in welcher

X die obenangegebene Bedeutung hat mit β-Dicarbonylverbindungen der allgemeinen Formel III

$$R^2-O-\overset{O}{\overset{\|}{C}}-CH_2-\overset{O}{\overset{\|}{C}}-R^1 \qquad (III)$$

in welcher

$R^1$ und $R^2$ die obenangegebene Bedeutung haben, und mit Enaminen der allgemeinen Formel IV

$$R^3-O-\overset{O}{\overset{\|}{C}}-CH=\overset{NHR}{\overset{|}{C}}-R^4 \qquad (IV)$$

in welcher

R, $R^3$ und $R^4$ die obenangegebene Bedeutung haben, gegebenenfalls in Gegenwart inerter organischer Lösungsmittel bei erhöhter Temperatur umsetzt oder

b) Ylidenverbindungen der allgemeinen Formel V

$$R^2-O-\overset{O}{\overset{\|}{C}}-\underset{R^1-\overset{|}{C}=O}{C}=\overset{X}{\overset{|}{C}H} \qquad (V)$$

in welcher

$R^1$, $R^2$ und X die obenangegebene Bedeutung haben, mit Enaminen der obenangegebenen Formel IV gegebenenfalls in Gegenwart von inerten organischen Lösungsmitteln umsetzt oder

c) zur Herstellung symmetrischer sila-substituierter 1,4-Dihydropyridin-Derivate Aldehyde der oben angegebenen Formel II mit der zweifachmolaren Menge siliciumhaltiger β-Dicarbonylverbindungen der allgemeinen Formel III

$$R^2-O-\overset{O}{\overset{\|}{C}}-CH_2-\overset{O}{\overset{\|}{C}}-R^1 \qquad (III)$$

in welcher der siliciumhaltige Rest $R^2$ und $R^1$ die obenangegebene Bedeutung haben,

und mit Aminderivaten der allgemeinen Formel VI

$$H_2N - R \qquad (VI)$$

in welcher

R die obenangegebene Bedeutung hat, gegebenenfalls in Gegenwart inerter organischer Lösungsmittel umsetzt.

4. Arzneimittel, dadurch gekennzeichnet, daß sie mindestens eine Verbindung aus der allgemeinen Formel I gemäß Anspruch 1 enthalten.

5. Verfahren zur Herstellung von Arzneimitteln, dadurch gekennzeichnet, daß man mindestens ein sila-substituiertes 1,4-Dihydropyridin-Derivat gemäß Anspruch 1 gegebenenfalls unter Verwendung von inerten organischen Hilfs- und Trägerstoffen in eine geeignete Applikationsform überführt.

6. Verbindungen der allgemeinen Formel I gemäß Anspruch 1 zur Verwendung bei der Heilung von Kreislauferkrankungen.

## Claims

1. Sila-substituted 1,4-dihydropyridine derivatives of the general formula I

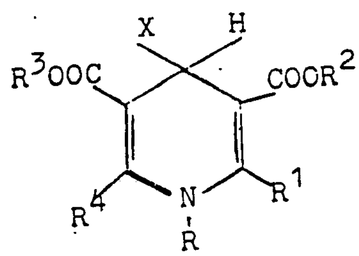

(I)

in which

R represents hydrogen, alkyl with 1 to 4 carbon atoms or benzyl,

$R^1$ and $R^4$ are identical or different and represent hydrogen, alkyl with 1 to 4 carbon atoms or trifluoromethyl,

$R^2$ and $R^3$ are identical or different and represent the radical

$$-A-\underset{R^5}{\underset{|}{\overset{R^7}{\overset{|}{Si}}}}-R^6$$

wherein

A denotes alkylene with 1 to 6 carbon atoms which is optionally substituted by alkyl with 1 to 4 carbon atoms and

$R^5$, $R^6$ and $R^7$ are identical or different and

each represent alkyl with 1 to 4 carbon atoms, cycloalkyl with 3 to 7 carbon atoms or phenyl, and these radicals are, in turn, optionally substituted by alkyl or alkoxy, each with 1 to 2 carbon atoms, halogen, in particular fluorine, chlorine or bromine, and trifluoromethyl,

or

one of the substituents $R^2$ or $R^3$ represents straight-chain or branched alkyl, cycloalkyl, alkenyl or alkinyl with up to 6 carbon atoms, and the alkyl radical, in turn, is optionally substituted by alkoxy with 1 to 4 carbon atoms, halogen, in particular fluorine, chlorine or bromine, or by a phenyl radical which in turn can carry 1 or 2 substituents from the group comprising trifluoromethyl, nitro, halogen, in particular fluorine, chlorine or bromine, and alkyl and alkoxy, each with 1 to 4 carbon atoms

or

one of the substituents $R^2$ and $R^3$ represents alkyl with 1 to 4 carbon atoms, which is substituted by the group

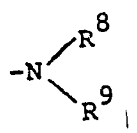

wherein

$R^8$ and $R^9$ are identical or different and each represent hydrogen, alkyl with 1 to 4 carbon atoms, phenyl or benzyl

and

X represents alkyl with 1 to 4 carbon atoms, cycloalkyl with 3 to 7 carbon atoms, cycloalkenyl with 5 to 7 carbon atoms, phenylalkyl with 1 to 3 carbon atoms in the alkyl chain or phenyl or naphthyl, and the phenyl radical is optionally substituted by 1, 2 or 3 identical or different substituents from the group comprising nitro, cyano, halogen, in particular fluorine, chlorine or bromine, trifluoromethyl, amino, trifluoromethoxy, alkyl, alkoxy, alkylmercapto or alkylsulphonyl with 1 to 4 carbon atoms in each of the alkyl and alkoxy radicals mentioned,

or represents a quinolyl, isoquinolyl, pyridyl, pyrimidyl, thienyl, furyl or pyrryl radical which is optionally substituted by halogen, in particular fluorine, chlorine or bromine, alkyl with 1 to 4 carbon atoms or alkoxy with 1 to 4 carbon atoms.

2. Compounds of the general formula I according to Claim 1

in which

R represents hydrogen or methyl,

$R^1$ and $R^4$ represent methyl,

$R^2$ and $R^3$ are identical or different and represent the radical

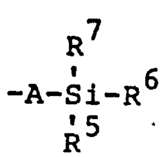

in which

A represents alkylene with 1 to 6 carbon atoms which is optionally substituted by methyl and

$R^5$, $R^6$ and $R^7$ are identical or different and each represent alkyl with 1 to 4 carbon atoms, cycloalkyl with 3 to 7 carbon atoms or phenyl, and these radicals are in turn optionally substituted by alkyl or alkoxy each with 1 to 2 carbon atoms, halogen, in particular fluorine, chlorine or bromine, and trifluoromethyl

or

one of the substituents $R^2$ or $R^3$ represents straight-chain or branched alkyl, cycloalkyl, alkenyl or alkinyl with up to 6 carbon atoms, and the alkyl radical, in turn, is optionally substituted by alkoxy with 1 to 4 carbon atoms, halogen, in particular fluorine, chlorine or bromine, or by a phenyl radical which in turn can carry 1 or 2 substituents from the group comprising trifluoromethyl, nitro, halogen, in particular fluorine, chlorine or bromine, and alkyl and alkoxy, each with 1 to 4 carbon atoms

or

one of the substituents $R^2$ and $R^3$ represents alkyl with 1 to 4 carbon atoms, which is substituted by the group

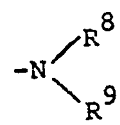

wherein

$R^8$ or $R^9$ are identical or different and each represent hydrogen, alkyl with 1 to 4 carbon atoms, phenyl or benzyl

and

X represents phenyl or naphthyl and the phenyl radical is optionally substituted by 1, 2 or 3 identical or different substituents from the group comprising nitro, cyano, halogen, in particular fluorine, chlorine or bromine, trifluoromethyl, amino, trifluoromethoxy, alkyl, alkoxy, alkylmercapto, or alkylsulphonyl each with 1 to 4 carbon atoms, or represents a quinolyl, isoquinolyl, pyridyl, pyrimidyl, thienyl, furyl or pyrryl radical which is optionally substituted by halogen, in particular fluorine, chlorine or bromine, alkyl with 1 to 4 carbon atoms or alkoxy with 1 to 4 carbon atoms.

3. A process for the production of sila-substituted 1,4-dihydropyridine derivatives of the general formula I

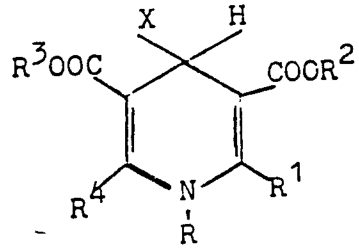

(I)

in which

R represents hydrogen, alkyl with 1 to 4 carbon atoms or benzyl,

$R^1$ and $R^4$ are identical or different and represent hydrogen, alkyl with 1 to 4 carbon atoms or trifluoromethyl,

$R^2$ and $R^3$ are identical or different and represent the radical

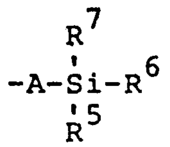

wherein

A denotes alkylene with 1 to 6 carbon atoms which is optionally substituted by alkyl with 1 to 4 carbon atoms and

$R^5$, $R^6$ and $R^7$ are identical or different and each represents alkyl with 1 to 4 carbon atoms, cycloalkyl with 3 to 7 carbon atoms or phenyl, and these radicals are, in turn, optionally substituted by alkyl or alkoxy, each with 1 to 2 carbon atoms, halogen, in particular fluorine, chlorine or bromine, and trifluoromethyl,

or

one of the substituents $R^2$ or $R^3$ represents straight-chain or branched alkyl, cycloalkyl, alkenyl or alkinyl with up to 6 carbon atoms, and the alkyl radical, in turn, is optionally substituted by alkoxy with 1 to 4 carbon atoms, halogen, in particular fluorine, chlorine or bromine, or by a phenyl radical which in turn can carry 1 or 2 substituents from the group comprising trifluoromethyl, nitro, halogen, in particular fluorine, chlorine or bromine, and alkyl and alkoxy, each with 1 to 4 carbon atoms.

or

one of the substituents $R^2$ and $R^3$ represents alkyl with 1 to 4 carbon atoms, which is substituted by the group

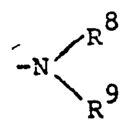

wherein

$R^8$ and $R^9$ are identical or different and each represent hydrogen, alkyl with 1 to 4 carbon atoms, phenyl or benzyl

and

X represents alkyl with 1 to 4 carbon atoms, cycloalkyl with 3 to 7 carbon atoms, cycloalkenyl with 5 to 7 carbon atoms, phenylalkyl with 1 to 3 carbon atoms in the alkyl chain, or phenyl or naphthyl, and the phenyl radical is optionally substituted by 1, 2 or 3 identical or different substituents from the group comprising nitro, cyano, halogen, in particular fluorine, chlorine or bromine, trifluoromethyl, amino, trifluoromethoxy, alkyl, alkoxy, alkylmercapto or alkylsulphonyl with 1 to 4 carbon atoms in each of the alkyl and alkoxy radicals mentioned,

or represents a quinolyl, isoquinolyl, pyridyl, pyrimidyl, thienyl, furyl or pyrryl radical which is optionally substituted by halogen, in particular fluorine, chlorine or bromine, alkyl with 1 to 4 carbon atoms or alkoxy with 1 to 4 carbon atoms,

characterised in that

a) aldehydes of the general formula II

$$X - CHO \qquad (II)$$

in which

X has the abovementioned meaning are reacted with β-dicarbonyl compounds of the general formula III

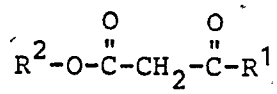

(III)

in which

$R^1$ and $R^2$ have the abovementioned meaning and with enamines of the general formula IV

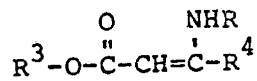

(IV)

in which

R, $R^3$ and $R^4$ have the abovementioned meaning, optionally in the presence of inert organic solvents at elevated temperature or

b) ylidene compounds of the general formula V

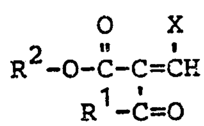

(V)

in which

$R^1$, $R^2$ and X have the abovementioned meaning, are reacted with enamines of the abovementioned formula IV, optionally in the presence of inert organic solvents or

c) to prepare symmetrical sila-substituted 1,4-dihydropyridine derivatives aldehydes of the abovementioned formula II are reacted with a two-fold molar amount of silicon-containing β-dicarbonyl compounds of the general formula III

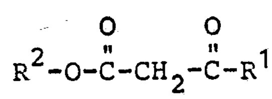

(III)

in which

the silicon-containing radical $R^2$ and

$R^1$ have the abovementioned meaning,

and with amine derivatives of the general formula VI

$$H_2N - R \qquad (VI)$$

in which

R has the abovementioned meaning, optionally in the presence of inert organic solvents.

4. Medicaments characterised in that they contain at least one compound of the general formula I according to Claim 1.

5. Process for the production of medicaments, characterised in that at least one sila-substituted 1,4-dihydropyridine derivative according to Claim 1 is converted into a suitable form of administration, optionally using inert organic au-

xiliaries and excipients.

6. Compounds of the general formula I according to Claim 1 for use in curing circulatory diseases.

**Revendications**

1. Dérivés de 1,4-dihydropyridine sila-substitués de formule générale I

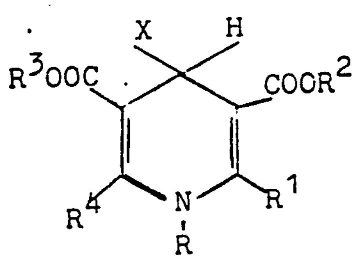

(I)

dans laquelle

R représente de l'hydrogène, un alcoyle ayant 1 à 4 atomes de carbone ou benzyle,

$R^1$ et $R^4$ sont identiques ou différents et représentent de l'hydrogène, alcoyle ayant 1 à 4 atomes de carbone ou trifluorométhyle,

$R^2$ et $R^3$ sont identiques ou différents et représentent le radical

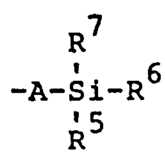

où

A représente un alcoylène ayant 1 à 6 atomes de carbone éventuellement substitué par un alcoyle ayant 1 à 4 atomes de carbone et

$R^5$, $R^6$ et $R^7$ sont identiques ou différents et représentent chaque fois un alcoyle ayant 1 à 4 atomes de carbone, cycloalcoyle ayant 3 à 7 atomes de carbone ou phényle, ces radicaux de leur côté étant substitués éventuellement par un alcoyle ou alcoxy ayant chacun 1 à 2 atomes de carbone, de l'halogène, en particulier du fluor, chlore ou brome et trifluorométhyle,

ou

un des substituants $R^2$ et $R^3$ représente un alcoyle à chaîne droite ou ramifiée, cycloalcoyle, alcényle ou alcinyle ayant jusqu'à 6 atomes de carbone, le radical alcoyle à son tour étant éventuellement substitué par un alcoxy ayant 1 à 4 atomes de carbone, de l'halogène, en particulier du fluor, chlore, brome, ou par un radical phényle qui, de son côté, peut porter 1 ou 2 substituants appartenant au groupe trifluorométhyle, nitro, halogène, en particulier fluor, chlore, brome, alcoyle et alcoxy ayant chacun 1 à 4 atomes de carbone,

ou

un des substituants $R^2$ et $R^3$ représente un alcoyle ayant 1 à 4 atomes de carbone, qui est substitué par le groupe

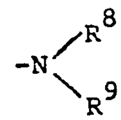

où

$R^8$ et $R^9$ sont identiques ou différents et représentent chaque fois de l'hydrogène, alcoyle ayant 1 à 4 atomes de carbone, phényle ou benzyle,

X représente un alcoyle ayant 1 à 4 atomes de carbone, cycloalcoyle ayant 3 à 7 atomes de carbone, cycloalcényle ayant 5 à 7 atomes de carbone, phénylalcoyle ayant 1 à 3 atomes de carbone dans la chaîne alcoyle, phényle ou naphtyle, le radical phényle étant éventuellement substitué par 1, 2 ou 3 substituants identiques ou différents appartenant au groupe nitro, cyano, halogène, en particulier fluor, chlore, brome, trifluorométhyle, amino, trifluorométhoxy, alcoyle, alcoxy, alcoylmercapto ou alcoylsulfonyle avec chaque fois 1 à 4 atomes de carbone dans les radicaux alcoyle et alcoxy cités,

ou un radical quinoléyle, isoquinoléyle, pyridyle, pyrimidyle, thiényle, furyle ou pyrryle éventuellement substitué par de l'halogène, en particulier du fluor, chlore, brome, alcoyle ayant 1 à 4 atomes de carbone ou alcoxy ayant 1 à 4 atomes de carbone.

2. Composés de formule générale I selon la revendication 1,

dans laquelle

R représente de l'hydrogène ou méthyle,

$R^1$ et $R^4$ représentent un méthyle,

$R^2$ et $R^3$ sont identiques ou différents et représentent le radical

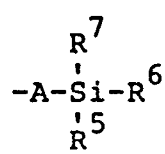

où

A représente un alcoylène ayant 1 à 6 atomes de carbone éventuellement substitué par un méthyle,

$R^5$, $R^6$ et $R^7$ sont identiques ou différents et représentent chacun un alcoyle ayant 1 à 4 atomes de carbone, cycloalcoyle ayant 3 à 7 atomes de carbone ou phényle, ces radicaux de leur côté étant éventuellement substitués par un alcoyle ou alcoxy ayant chacun 1 à 2 atomes de carbone, de l'halogène, en particulier du fluor, chlore ou brome, et trifluorométhyle,

ou

un des substituants $R^2$ ou $R^3$ représente un alcoyle à chaîne droite ou ramifiée, cycloalcoyle, alcényle ou alcinyle ayant jusqu'à 6 atomes de carbone, le radical alcoyle de son côté étant substitué éventuellement par un alcoxy ayant 1 à 4 atomes de carbone, de l'halogène, en particulier du fluor, chlore, brome, ou par un radical phényle qui, de son côté, peut porter 1 ou 2 substituants appartenant au groupe trifluorométhyle, nitro, halogène, en particulier fluor, chlore, brome, alcoyle et alcoxy ayant chacun 1 à 4 atomes de carbone,

ou

un des substituants $R^2$ et $R^3$ représente un alcoyle ayant 1 à 4 atomes de carbone qui est substitué par le groupe

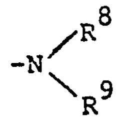

qui sont identiques ou différents et représentent chacun de l'hydrogène, alcoyle ayant 1 à 4 atomes de carbone, phényle ou benzyle et

X représente un phényle ou naphtyle, le radical phényle étant éventuellement substitué par 1, 2 ou 3 substituants identiques ou différents appartenant au groupe nitro, cyano, halogène, en particulier fluor, chlore, brome, trifluorométhyle, amino, trifluorométhoxy, alcoyle, alcoxy, alcoylmercapto ou alcoylsulfonyle ayant chaque fois 1 à 4 atomes de carbone, ou un radical quinoléyle, isoquinoléyle, pyridyle, pyrimidyle, thiényle, furyle ou pyrryle éventuellement substitué par de l'halogène, en particulier fluor, chlore, brome, alcoyle ayant 1 à 4 atomes de carbone ou alcoxy ayant 1 à 4 atomes de carbone.

3. Procédé de préparation de dérivés de 1,4-dihydropyridine sila-substitués de formule générale I

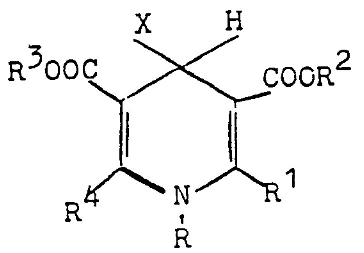

(I)

dans laquelle

R représente de l'hydrogène, alcoyle ayant 1 à 4 atomes de carbone ou benzyle,

$R^1$ et $R^4$ sont identiques ou différents et représentent de l'hydrogène, alcoyle ayant 1 à 4 atomes de carbone ou trifluorométhyle,

$R^2$ et $R^3$ sont identiques ou différents et représentent le radical

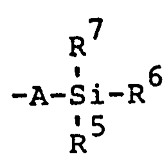

où

A représente un alcoylène ayant 1 à 6 atomes de carbone éventuellement substitué par un alcoyle ayant 1 à 4 atomes de carbone et

$R^5$, $R^6$ et $R^7$ sont identiques ou différents et représentent chaque fois un alcoyle ayant 1 à 4 atomes de carbone, cycloalcoyle ayant 3 à 7 atomes de carbone ou phényle, ces radicaux de leur côté étant éventuellement substitués par un alcoyle ou alcoxy ayant chacun 1 à 2 atomes de carbone, de l'halogène, en particulier du fluor, chlore ou brome et trifluorométhyle,

ou

un des substituants $R^2$ ou $R^3$ représente un alcoyle à chaîne droite ou ramifiée, cycloalcoyle, alcényle ou alcinyle ayant jusqu'à 6 atomes de carbone, le radical alcoyle de son côté étant éventuellement substitué par un alcoxy ayant 1 à 4 atomes de carbone, de l'halogène, en particulier du fluor, chlore, brome ou par un radical phényle qui, à son tour, peut porter 1 ou 2 substituants appartenant au groupe trifluorométhyle, nitro, halogène, en particulier fluor, chlore, brome, alcoyle et alcoxy ayant chacun 1 à 4 atomes de carbone,

ou

un des substituants $R^2$ et $R^3$ représente un alcoyle ayant 1 à 4 atomes de carbone qui est substitué par le groupe

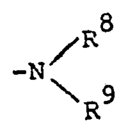

où $R^8$ et $R^9$ sont identiques ou différents et représentent chacun de l'hydrogène, alcoyle ayant 1 à 4 atomes de carbone, phényle ou benzyle et

X représente un alcoyle ayant 1 à 4 atomes de carbone, cycloalcoyle ayant 3 à 7 atomes de carbone, cycloalcényle ayant 5 à 7 atomes de carbone, phénylalcoyle ayant 1 à 3 atomes de carbone dans la chaîne alcoyle, phényle ou naphtyle, le radical phényle étant éventuellement substitué par 1, 2 ou 3 substituants identiques ou différents appartenant au groupe nitro, cyano, halogène, en particulier fluor, chlore, brome, trifluorométhyle, amino, trifluorométhoxy, alcoyle, alcoxy, alcoylmercapto ou alcoylsulfonyle ayant chaque fois 1, 4 atomes de carbone dans les radicaux alcoyle et alcoxy cités,

ou représente un radical quinoléyle, isoquinoléyle, pyridyle, pyrimidyle, thiényle, furyle ou pyrryle éventuellement substitué par de l'halogène, en particulier du fluor, chlore, brome, alcoyle ayant 1 à 4 atomes de carbone ou alcoxy ayant 1 à 4 atomes de carbone,

caractérisé en ce qu'on fait réagir

a) des aldéhydes de formule générale II

$$X - CHO \quad (II)$$

dans laquelle

X a la signification indiquée plus haut, avec des composés β-dicarbonylés de formule générale III

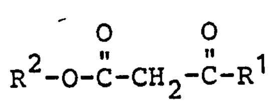

(III)

dans laquelle

$R^1$ et $R^2$ ont la signification indiquée plus haut, et avec des énamines de formule générale IV

$$R^3-O-\overset{O}{\overset{\|}{C}}-CH=\overset{NHR}{\overset{|}{C}}-R^4 \qquad (IV)$$

dans laquelle

R, $R^3$ et $R^4$ ont la signification indiquée plus haut, éventuellement en présence de solvants organiques inertes à température élevée, ou en ce qu'on fait réagir

b) des composés ylidène de formule générale V

$$R^2-O-\overset{O}{\overset{\|}{C}}-\underset{R^1-\overset{|}{C}=O}{C}=\overset{X}{\overset{|}{CH}} \qquad (V)$$

dans laquelle

$R^1$, $R^2$ et X ont la signification indiquée plus haut, avec des énamines de formule IV indiquée plus haut éventuellement en présence de solvants organiques inertes, ou

c) pour la préparation de dérivés de 1,4-dihydropyridine sila-substitués symétriques, on fait réagir des aldéhydes de formule II indiquée plus haut avec la quantité doublement molaire de composés β-dicarbonylés contenant du silicium de formule générale III

$$R^2-O-\overset{O}{\overset{\|}{C}}-CH_2-\overset{O}{\overset{\|}{C}}-R^1 \qquad (III)$$

dans laquelle

le radical renfermant du silicium $R^2$ et $R^1$ ont la signification indiquée plus haut,

et avec des dérivés aminés de formule générale VI

$$H_2N - R \qquad (VI)$$

dans laquelle

R a la signification indiquée plus haut, éventuellement en présence de solvants organiques inertes.

4. Médicaments, caractérisés en ce qu'ils contiennent au moins un composé de formule générale I selon la revendication 1.

5. Procédé de préparation de médicaments, caractérisé en ce qu'on convertit au moins un dérivé de 1,4-dihydropyridine sila-substitué selon la revendication 1, éventuellement avec utilisation de substances auxiliaires et de support organiques inertes, en une forme d'application adéquate.

6. Composés de formule générale I selon la revendication 1 pour l'utilisation dans la guérison de maladies de la circulation.